# EUROPEAN PATENT APPLICATION

(11) **EP 4 725 483 A1**
(43) Date of publication of application: **15.04.2026**
(21) Application number: 24306680.0
(22) Date of filing: 14.10.2024
(51) Int. Cl.: A61K 31/439, A61K 31/517, A61K 31/445, A61K 45/06, A61P 25/02, A61P 25/16, A61K 31/13, A61K 31/135, A61K 31/15, A61K 31/166, A61K 31/195, A61K 31/197, A61K 31/198, A61K 31/335, A61K 31/381, A61K 31/4045, A61K 31/407, A61K 31/4178, A61K 31/4184, A61K 31/428, A61K 31/437, A61K 31/438, A61K 31/4468

(54) **COMBINATION COMPRISING AT LEAST ONE 5-HT2A RECEPTOR ANTAGONIST AND AT LEAST ONE 5-HT3 RECEPTOR ANTAGONIST AND ITS MEDICAL USE**

(71) Applicant: Université de Bordeaux, 33000 Bordeaux (FR); Institut National de la Santé et de la Recherche Médicale, 75013 Paris (FR); Centre National de la Recherche Scientifique, 75016 Paris (FR)
(72) Inventor: Fossat, Pascal, 33600 Pessac (FR); Aby, Franck, 33000 Bordeaux (FR); Benazzouz, Abdelhamid, 33600 Pessac (FR)
(74) Representative: Novagraaf Technologies

(57) **Abstract**

The present invention relates to a combination comprising at least one 5-HT2A receptor antagonist and at least one 5-HT3 receptor antagonist.

The present invention also relates to a pharmaceutical composition comprising at least one 5-HT2A receptor antagonist and at least one 5-HT3 receptor antagonist, and optionally at least one other active ingredient.

The present invention to a kit of parts comprising at least one 5-HT2A receptor antagonist and at least one 5-HT3 receptor antagonist as a combined preparation for simultaneous, separate or sequential use, and to the combination or the pharmaceutical composition of the invention for medical use.

## Description

### Technical field

The present invention refers to a combination comprising HT receptor antagonists, especially for medical use, and to pharmaceutical compositions.

Therefore, the present invention has utility in medical field.

In the description below, the references into brackets ([ ]) refer to the listing of references situated at the end of the text.

### Background of the Invention

Parkinson's disease is the second most common neurodegenerative disease affecting 2-3% of the population over the age of 60-65, an estimated 0.3% of the general population. It is identified as the fastest growing neurological disorder in terms of deaths and disability. Parkinson's disease is well-known for motor symptoms, consequence of a progressive neurodegeneration notably altering dopamine (DA) neurons of the substantia nigra pars compacta (SNc). Parkinson's disease is also characterized by non-motor symptoms that appear even before motor deficits, and worsen as the disease progresses. Among these non-motor symptoms, pathological pain is one of the more prominent and debilitating affecting up to 95% of patients with Parkinson's disease who qualified it as one of the most troublesome non-motor symptoms throughout the disease.

Current treatments to relieve pain in Parkinson's disease patients depend on the type of pain but have mainly poor efficacy. The high prevalence of chronic pain in Parkinson's disease and the lack of efficient therapies appeal to a better understanding of the underlying mechanisms.

Thus, a need exists of alternative tools for use in the treatment or prevention of non-motor component of Parkinson's disease, especially Parkinson's disease-associated pain.

### Description of the invention

Thanks to extensive *in vivo* research work, the inventors surprisingly found that 5-HT neurons in the NRM are hyperactive and mediate descending facilitation promoting spinal hyperexcitability and pain hypersensitivity through 5-HT_{2A} and 5-HT₃ receptors in Parkinson's disease.

Indeed, the inventors showed that serotonin (5-HT) levels increased in the spinal cord, correlating with reduced tyrosine hydroxylase (TH) immunoreactivity in the nucleus raphe magnus (NRM) and increased excitability of 5-HT neurons. They also showed that selective optogenetic inhibition of 5-HT neurons attenuated mechanical hypersensitivity and reduced DHSC hyperexcitability. In addition, blockade of 5-HT_{2A} and 5-HT₃ receptors reduced mechanical hypersensitivity.

These results reveal, for the first time, that PD-like dopamine depletion triggers spinal-mediated mechanical hypersensitivity, associated with serotonergic hyperactivity in the NRM.

Accordingly, in a first aspect, the present invention provides a combination comprising at least one 5-HT_{2A} receptor antagonist and at least one 5-HT₃ receptor antagonist.

"5-HT_{2A} receptor antagonist" refers herein to any compound that can block 5-HT_{2A} receptor. It may be for example at least one 5-HT_{2A} receptor antagonist selected in the group consisting of glemanserin, ketanserin, volinanserin, altanserin, ritanserin, clozapine, loxapine, olanzapine, aripiprazole, quetiapine, ziprasidone, asenapine, amitriptyline, clomipramine, cyproheptadine, doxepin, eplivanserin, etoperidone, haloperidol, hydroxyzine, iloperidone, methysergide, mianserine, mirtazapine, nefazodone, pimavanserin, pizotifen, trazodone, yohimbine, spiperone, pirenperone and risperidone. Preferably, the antagonist may be a selective antagonist. Selective 5-HT_{2A} receptor antagonist may be for example at least one selected in the group consisting of glemanserin, ketanserin, volinanserin, altanserin and pimavanserin.

"5-HT₃ receptor antagonist" refers herein to any compound that can block 5-HT₃ receptor. It may be for example at least one 5-HT₃ receptor antagonist selected in the group consisting of granisetron, ondansetron, tropisetron, alosetron, dolasetron, palonosetron, ramosetron, memantine, metoclopramide and vortioxetine. Preferably, the antagonist may be a selective antagonist. Selective 5-HT₃ receptor antagonist may be for example at least one selected in the group consisting of granisetron, ondansetron, tropisetron, alosetron, dolasetron, palonosetron and ramosetron.

In a preferred embodiment, 5-HT_{2A} receptor antagonist is glemanserin or ketanserin, and said 5-HT₃ receptor antagonist is granisetron.

"Combination" refers herein to a formulation wherein the at least one 5-HT_{2A} receptor antagonist and the at least one 5-HT₃ receptor antagonist show synergistic effects in reducing or preventing of non-motor component of Parkinson's disease, especially Parkinson's disease-associated pain. The term "synergy" or "synergistic" as used herein refers to a therapeutic combination which is more effective than the additive effects of the two single kinds of active agents, i.e. at least one 5-HT_{2A} receptor antagonist and at least one 5-HT₃ receptor antagonist. A determination of a synergistic interaction between these active agents may be based on the results obtained from the assays described herein. The results of these assays may be analyzed using the simplified up and down method for mechanical pain threshold assay.

According to the invention, a synergistic effect may be attained when the active agents are: (1) co-formulated and administered or delivered simultaneously in a combined, unit dosage formulation; or (2) delivered as separate formulations. When delivered in separate formulations, a synergistic effect may be attained when the compounds are administered or delivered simultaneously or sequentially, for example by different injections in separate syringes. In this case, the combination may be administered in two or more administrations, for example in consecutive administration in either order, wherein preferably there is a time period while both (or all) active agents simultaneously exert their biological activities.

Whether by coadministration or by simultaneous, separate or sequential regimen, a therapeutically effective amount of each active ingredient may be administered. As an example, a pharmaceutically effective amount of at least one 5-HT_{2A} receptor antagonist may be in the range of about 10 mg to 40 mg per day, depending on the route of administration. Also as an example, a pharmaceutically effective amount of at least one 5-HT₃ receptor antagonist may be in the range of about 1 mg to about 5 mg per day, depending on the route of administration.

According to the invention, "at least one" refers to 1, or 2, or 3, or 4, or more of the cited active agents.

Another object of the invention relates to a pharmaceutical composition comprising at least one 5-HT_{2A} receptor antagonist and at least one 5-HT₃ receptor antagonist as defined above, and optionally at least one other active ingredient. The pharmaceutical composition may be formulated in accordance with standard pharmaceutical practice for use in a therapeutic treatment in mammals, including humans. Therefore, pharmaceutical compositions of the present invention include combinations of active agents as described above, and one or more pharmaceutically acceptable carrier, diluent, or excipient. The phrase "pharmaceutically acceptable" indicates that the substance or composition must be compatible chemically and/or toxicologically, with the other ingredients of the formulation, and the mammal being treated therewith. Suitable carriers, diluents and excipients are well known to those skilled in the art and include materials such as carbohydrates, waxes, water soluble and/or swellable polymers, hydrophilic or hydrophobic materials, gelatin, oils, solvents, water and the like. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, saline, cyclodextrin, ethanol, propylene glycol, polyethylene glycols (e.g., PEG 400, PEG 300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents and flavoring agents.

The pharmaceutical compositions of the invention may be prepared using conventional dissolution and mixing procedures. For example, the bulk drug substance may be dissolved in a suitable solvent in the presence of one or more of the excipients described above.

The pharmaceutical compositions of the invention may be administered by any route appropriate to the condition to be treated, which includes oral and parenteral routes, the latter including subcutaneous, intramuscular, intravenous, intraarterial, intradermal, intrathecal, epidural, and infusion techniques. It will be appreciated that the preferred route may vary with for example the condition of the recipient. Where the composition is administered orally, it may be formulated as tablets, pills, hard or soft e.g., gelatin capsules, cachets, troches, lozenges, aqueous or oil suspensions, dispersible powders or granules, emulsions, syrups or elixirs each containing a predetermined amount of each active agent, with a pharmaceutically acceptable carrier, glidant, or excipient. Where the composition is administered parenterally, it may be formulated with a pharmaceutically acceptable parenteral vehicle or diluent, and in a unit dosage injectable form, as detailed below. For example, formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

Advantageously, the at least one active ingredient is selected in the group consisting of L-Dopa, a dopamine agonist such as apomorphine, pramipexole, ropinirole or rotigotine, antidepressants such as serotonin noradrenaline reuptake inhibitors (SNRI) or tricyclic antidepressants (TCA), and GABAergic agents such as pregabaline or gabapentine.

Another object of the invention relates to a kit of parts comprising at least one 5-HT_{2A} receptor antagonist and at least one 5-HT₃ receptor antagonist as defined above, as a combined preparation for simultaneous, separate or sequential use. All the definitions given above are likely to apply mutatis mutandis to the kit of parts of the invention.

The "kit" according to the invention may be provided as an article of manufacture containing the active agents useful for the treatment of the diseases and disorders described above. The kit may comprise one container per active agent, or one container containing all the active agents. The kit may further comprise a label or package insert, on or associated with the container. The term "package insert" is used to refer to instructions customarily included in commercial packages of therapeutic products, that contain information about directions for the administration, the indications, usage, dosage, administration, contraindications and/or warnings concerning the use of such therapeutic products. Especially, if the kit comprises a first formulation comprising at least one 5-HT_{2A} receptor antagonist and a second formulation comprising at least one 5-HT₃ receptor antagonist, the kit may further comprise directions for the simultaneous, sequential or separate administration of the two formulations to a patient in need thereof.

Another object of the invention relates to the combination as defined above, or the pharmaceutical composition as defined above, for medical use. More particularly, the medical use may be the treatment or prevention of non-motor component of Parkinson's disease, such as increased chronic pain.

This invention is further illustrated by the following examples with regard to the annexed drawings that should not be construed as limiting.

### Brief description of the figures

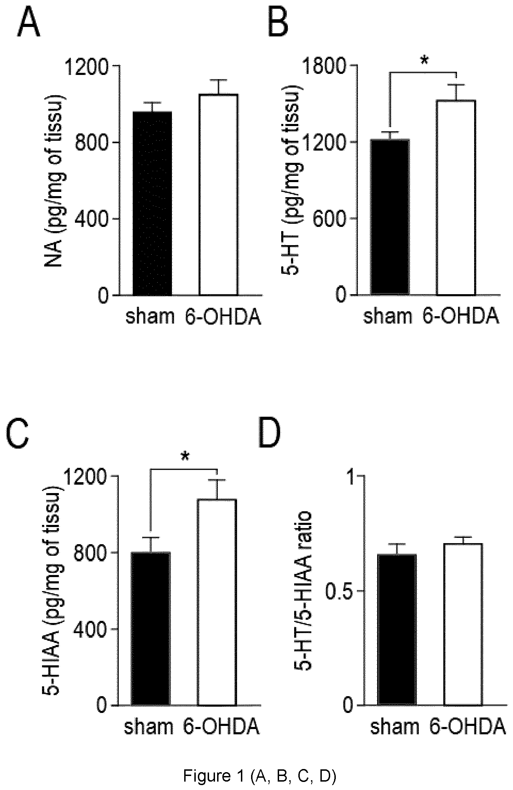

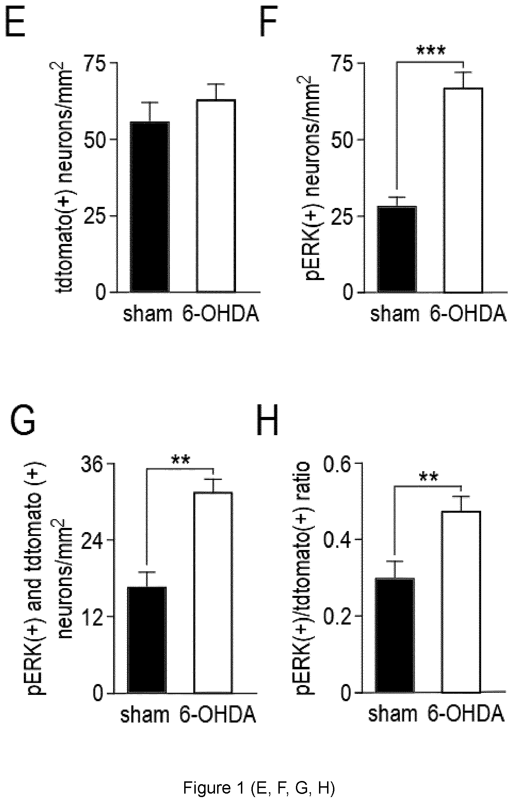

- Figure 1: The activity of serotonergic neurons is increased in the mice model of hemiparkinsonism. **(A)** Summary data comparing the HPLC (high performance liquid chromatography) dosage of NA (noradrenaline) from lumbar spinal cord of sham and 6-OHDA mice (962 ± 49 pg/mg, *n*=6 and 1053 ± 75 pg/mg, *n*=6, respectively). **(B)** Summary data comparing the HPLC dosage of 5-HT from lumbar spinal cord of sham and 6-OHDA mice (1222 ± 62 pg/mg, *n*=6 and 1528 ± 123 pg/mg, *n*=6, respectively). **(C)** Summary data comparing the HPLC dosage of 5-HIAA (5-hydroxyindolacetic acid) from lumbar spinal cord of sham and 6-OHDA mice (801 ± 82 pg/mg, *n*=6 and 1079 ± 105 pg/mg, *n*=6, respectively). **(D)** Summary data comparing the ratio between 5-HT and 5-HIAA in lumbar spinal cord of sham and 6-OHDA mice (0.65 ± 0.05, *n*=6 and 0.71 ± 0.03, *n*=6, respectively). **(E)** Summary data comparing tdtomato positive NRM neurons in sham and 6-OHDA mice (56.1 ± 6.5, *n*=9 and 63.2 ± 5.5, *n*=12, respectively). **(F)** Summary data comparing pERK positive NRM neurons in sham and 6-OHDA mice (28.6 ± 2.8, *n*=10 and 67.1 ± 5.1, *n*=13, respectively). **(G)** Summary data comparing both tdtomato and pERK positive NRM neurons in sham and 6-OHDA mice (16.7 ± 2.4, *n*=10 and 31.6 + 2.1, *n*=13, respectively). **(H)** Summary data comparing the specific ratio of tdtomato positive NRM neurons also positive pERK in sham and 6-OHDA mice (0.3 ± 0.04, *n*=10 and 0.47 ± 0.04, *n*=13, respectively).

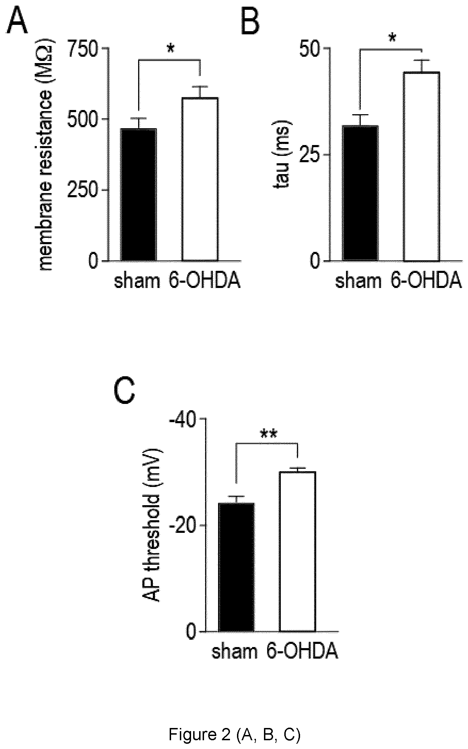

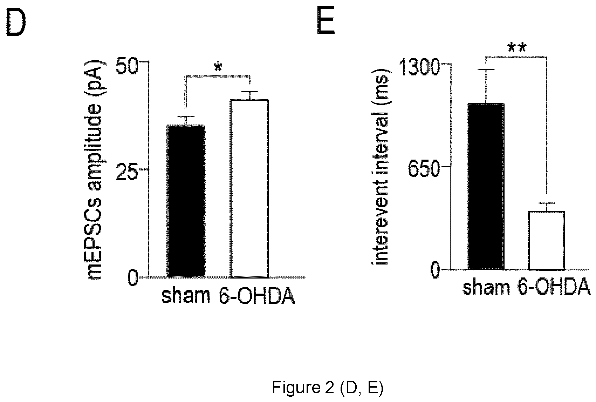

- Figure 2: Both neuronal excitability and excitatory inputs onto 5-HT neurons of the NRM are increased in the mice model of hemiparkinsonism. **(A)** summary data comparing the membrane resistance of NRM 5-HT neurons from sham and 6-OHDA mice (471 ± 37 MΩ, *n*=12 and 579 ± 42 MΩ, *n*=18, respectively). **(B)** summary data comparing the membrane capacitance of NRM 5-HT neurons from sham and 6-OHDA mice (tau value: 31 ± 2.4 ms, *n*=13 and 43.4 ± 3.0 ms, *n*=18, respectively). **(C)** summary data comparing the action potential threshold of NRM 5-HT neurons from sham and 6-OHDA mice (-25.7 ± 1.4 mV, *n*=13 and -31.7 ± 0.8 mV, *n*=18, respectively). **(D)** Average mEPSCs amplitude recorded in NRM 5-HT neurons from sham and 6-OHDA mice in the presence of TTX (tetrodotoxin, 1 µM). (35.5 ± 2.1 pA, *n*=16) and 6-OHDA (41.5 ± 1.9 pA, *n*=13) mice. **(G)** Average interevent intervals of mEPSCs of NRM 5-HT neurons from sham and 6-OHDA mice. (1051 ± 212 ms, *n*=16) and 6-OHDA (370 ± 56 pA, *n*=13) mice.

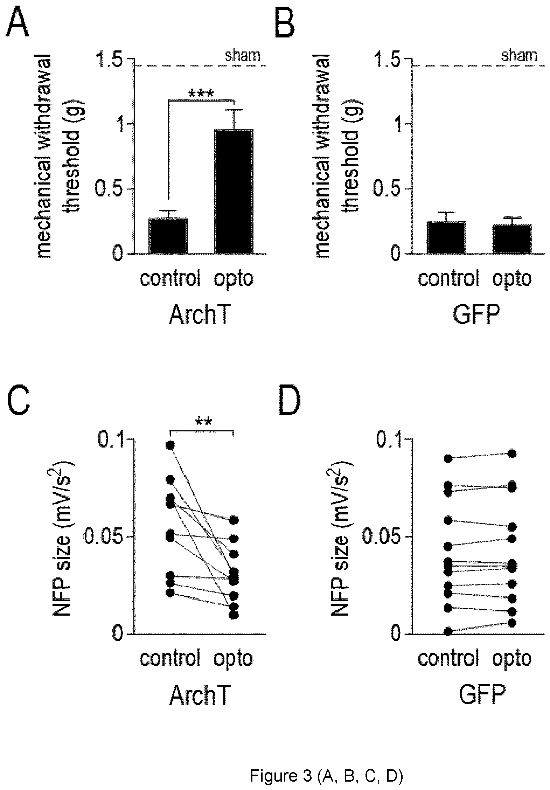

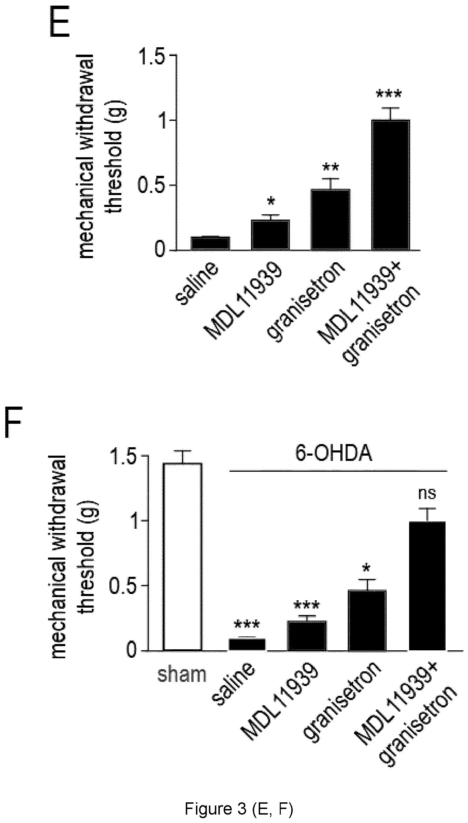

- Figure 3: Inhibition of NRM 5-HT neurons reverses mechanical hypersensitivity in the mice model of hemiparkinsonism. **(A)** Summary data comparing the effect of the optogenetic ArchT stimulation on the mechanical sensitivity of 6-OHDA mice (control: 0.27 ± 0.06 g vs. opto: 0.95 ± 0.16 g, *n*=15). The dashed line indicates the mechanical sensitivity of sham mice. **(B)** Same protocol as in (B) except that the expression of ArchT is replaced by GFP. Control: 0.26 ± 0.07 g vs. opto: 0.23 ± 0.05 g, *n*=12). **(C)** Summary data comparing the effect of the ArchT optogenetic stimulation on the amplitude of the nociceptive field potentials in 6-OHDA mice (control: 0.056 ± 0.008 mV/s² vs. opto: 0.031 ± 0.005 mV/s², *n*=10). **(D)** Summary data comparing the effect of the GFP optogenetic stimulation on the amplitude of the nociceptive field potentials in 6-OHDA mice (control: 0.042 ± 0.008 mV/s² vs. opto: 0.043 ± 0.008 mV/s², *n*=12). **(E)** Summary data comparing the effect of the intrathecal injection of 5-HT receptors antagonists on the mechanical sensitivity of 6-OHDA mice (saline: 0.11 ± 0.01 g, *n*=8; MDL11939: 0.24 ± 0.04 g, *n*=8; granisetron: 0.47± 0.09 g, *n*=8; MDL11939+gransitron: 1.01± 0.09 g, *n*=8). **(F)** same experiments as in (G) comparing the mechanical sensitivity of 6-OHDA mice in the presence of 5-HT receptors antagonists to sham mice (1.44 ± 0.09 g, *n*=29).

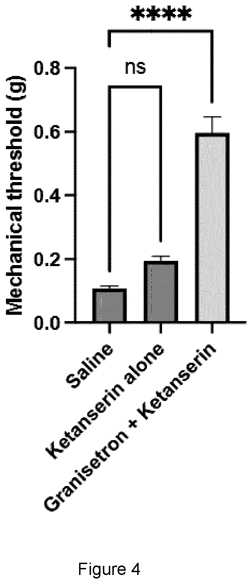

- Figure 4: shows mechanical hypersensitivity in mice after 6-OHDA injection in the mid forebrain bundle. The mechanical threshold (g) is measured after intrathecal injection in mice of saline (10µl), ketanserin alone (20 µg in 10µl), and ketanserin + granisetron (20 µg in 10µl for ketanserin and 14 µg in 10µl for granisetron). Only the combination of granisetron + ketanserin improves mechanical threshold.

### Examples

### Example 1: new combination for Parkinson's disease-associated pain targeting the serotonergic systems

### METHODS

### Animals

Adult (20g-25g) female and male wild-type (C57BI6/J), and transgenic *ePet-cre* (B6.Cg-Tg (FeV-cre) 1Esd/J, Jackson Laboratory) mice were used in this study. *ePet-cre* mice were crossbreed with Ai9tdtomato line mice (Gt(Rosa)26Sortm6(CAG-tdTomato)Hze, Jackson lab, USA), in order to obtain *ePet-cre::Ai9tdtomato* mice. They were housed in the EOPS animal facility with free access to food and water, in constant temperature (21±2 °C) and humidity levels (60%), under a 12:12 light/dark cycle, in a small social group setting (2-5 mice). All procedures were validated by the national and the local ethic committee of Bordeaux (CE50; Animal Facility PIV-EXPE, APAFIS#25605, APAFIS#32137 and APAFIS#26108; Animal Facilities of Neurocentre Magendie, APAFIS#21068). Animals were first submitted to surgical procedure to inject 6-OHDA or saline. In a first set of experiments, a group of 26 shams and 29 6-OHDA mice was used. 3 weeks after injection, animals were submitted to motor and sensory tests (see below). Then, either *in vivo* or patch-clamp electrophysiology was performed in the following days. Tissues were then extracted for immunohistochemistry. A group of 27 *ePet-cre* 6-OHDA mice was used for optogenetic (15 ArchT and 12 GFP).

### Surgery

Mice were anaesthetized in an induction chamber (isoflurane, 4%) then were placed into a stereotaxic frame (RWD, Shengzen, China) on a heating pad, head-fixed with ear bars and maintained at 1.5-2% isoflurane. Mice received ocular gel, betadine and a subcutaneous injection of buprenorphine (100 µL, 0.1 mg/kg) and local injection of lidocaine (100 µL, 0,4mg/kg).

### → 6-OHDA model

6-hydroxydopamine (6-OHDA) or vehicle (NaCl 0.9%) were injected into the right medial forebrain bundle (MFB). 6-OHDA (Sigma, Saint-Quentin Fallavier, France) 12.5 mg in 2.5 ml of a solution of 0.09% NaCl containing 1% ascorbic acid used within 12h. 1 µL of toxin was injected the MFB at the following coordinates (relative to bregma): AP= -1.2; ML =-1.3 and DV= from -4.85 to -4.65. An intraperitoneal injection of desipramine (Sigma Aldrich, France) at 25mg/kg was proceed 30 min before the 6-OHDA injection. Post-surgery care was necessary during the first three weeks after the surgery.

### → AAV injections

In parallel, we proceed to stereotaxic injections of virus needed for the expression of the Archaerhodopsin (ArchT) or Green Fluorescent Protein (GFP) in the nucleus raphe magnus (NRM). We injected a total of 600 nL (in 3 bolus of 200 nL, at coordinates from bregma AP/ML/DV - 5,6/0/-5,6; -5,8/0/-5,6; -6,1/0/-5,7), of selected viruses: AAV-CAG-Flex-ArchT-YFP (4,2×10¹² p/mL, UNC Vector Core) or AAV-CAG-Flex-GFP (8×10 12 p/ml, UNC Vector Core).

### → Spinal implantation of optic fibers

Optical cannulae (KFP2301LZXX LC Ceramic Ferule NO flange OD 1,25mm - ID 230 micron - conc < 20 micron from AMS Technologies, associated with ∅200 µm Core TECS-Clad Multimode Optical Fiber, 0.39 NA from Thorlabs) were implanted above the surface of the DHSC.

Spinal cord implantations followed the protocol described in Christensen et al. ([2]) in anesthetized mice (see above surgery). A 1- to 2-cm incision was made slightly caudal to the peak of the dorsal hump to expose the lumbar spinal region. The T12 L1 vertebra of interest was identified, and then a small incision was made between the tendons and the vertebral column on both sides. The vertebra was then secured using spinal adaptor clamps, and tissues were removed from the surface of the bone. A small hole was drilled approximately 1 mm from midline, centrally on the rostro-caudal axis on the left side. Finally, we sutured and cleansed the skin surrounding the dental cement securing the cannula implantation.

### Behavior experiments

For all behavioral experiments, mice were tested in the morning between 8h and 12h am. Experimenter was blinded from the animal group or treatment.

### Motor behavior experiments

Locomotor symptoms induced by 6-OHDA were evaluated using cylinder test adapted from Boix et al. ([3]). First, mice were placed individually inside a glass cylinder (diameter 144 mm). Each session of 2 min was videotaped and analysed using EthoVision^{®} software (XT, Noldus Information Technology, NL). The spontaneous rotation preference has been assessed by counting the number of clockwise (ipsilateral) and anticlockwise (controlateral) rotations.

Second, we placed the mice in an openfield of 40 × 40 cm for 10 min. Using the tracking Ethovision software, we evaluated the distance travelled by the animals.

### Von Frey tests

To assess the mechanical thresholds, we used a set of Von Frey filaments of different forces (UgoBasile, Italy). Prior testing, mice were habituated 30min to the testing apparatus. Von Frey filaments of different forces were applied to the plantar surface of the hind-paws. The simplified up-down method (SUDO) in which only 5 trials are done in the hind-paws, have been used (Bonin et al. ([4])). In case of paw withdrawal, we used the next lowest filament. The last filament used, indicates the threshold weighted by the value of the 4^{th} test. We converted the paw withdrawal threshold (PWT) measured to force (g) with the following equation: PWT_{force} = 10^{(x*F+B)} with PWT being the paw withdrawal threshold in grams used in the figures, F the filament number, x and B for filament numbered 2-7, x=0.240 and B=-2.00, and for the filament numbered 7-14, x=0.182 and B=-1.47.

We connected the 577nm laser to the optical fiber, positioned at the lumbar spinal cord level, with its power set at 10 mW/mm². Continuous stimulation of 3 min long were delivered.

### Pharmacological experiments

### → Intrathecal injection of 5-HT antagonists

To test the different antagonists, we inject a bolus of 10µL of solution containing the drug or only saline as control directly at the level of the spinal cord via an intrathecal injection with a 10µL Hamilton syringe equipped with a 30G needle. Drug are all dissolved in NaCl 0.9% solution.

The concentration of the drug and the time let between the injection and the behavioral measure can be found in the following table:

| **Antagonist** | **Reference** | **Concentration** | **Quantity** | **Time after injection** |
|---|---|---|---|---|
| MDL11939 (glemanserin) | Millipore (SML0115) | 0.1mM | 0.3µg | 30 min |
| Granisetron | Tocris (2903) | 4mM | 14 µg | 1h |

Granisetron and MDL11939 (glemanserin) have been injected separately to respect their time before behavioral measure.

### High Performance Liquid Chromatography (HPLC)

The ipsilateral and contralateral lumbar spinal cord were rapidly extracted and frozen in cold isopentane (-35°C). Tubes containing the lumbar cord samples were placed on ice, and rapidly wiped and weighed. Samples were then homogenized in 0.1N HClO4 (100 µL), sonicated and centrifuged at 13,000 rpm for 30 min at 4°C. The supernatants were injected into the HPLC system using a manual injector (Rheodyne 7725i, C.I.L. Cluzeau, Sainte-Foy-La-Grande, France) equipped with a 20 µL loop into the HPLC column (Hypersyl C18, 150x4.6 mm, 5 µm, C.I.L. Cluzeau) preceded by a Brownlee-Newgard precolumn (RP-8, 15×32 mm, 7 µm, C.I.L. Cluzeau). The filtered (0.22 mm Millipore filter) mobile phase was composed of (in mM): 60 NaH2PO4, 0.1 Disodium EDTA, 2-Octane-Sulfonic acid in deionized water (18MW cm2) containing 7% methanol. The mobile phase was delivered by an HPLC pump (LC-20-AD, Shimadzu, France) at 1.2 mL/min. Monoamine detection was obtained with a coulometric cell (5011 coulometric cell, ESA, Paris, France) coupled to a programmable detector (Coulochem II, ESA). The potential of the electrodes was set to -270 and +350 mV. Signals were acquired using an Ulyss interface and stored on a computer using dedicated software (Azur, Toulouse, France). Moreover, a standard solution (1 ng/10 µL) was administered before and after each daily series of sample analysis. Results are expressed in pg/mg of tissue.

### Electrophysiological experiments

### → Patch clamp experiments

The brain was rapidly extracted and immerged in cold sucrose artificial cerebral spinal fluid (aCSF) solution composed of (in mM) : 2 KCl, 26 NaHCOs, 1.15 NaH₂PO₄, 6 MgCl₂, 0.2 CaCl₂, 10 glucose, 220 sucrose, saturated with 95 % O₂-5 % CO₂. The brainstem is then separated from the rest of the brain and placed in the vibratome (LeicaVT1 100S) intercalated between two blocks of 4 % agarose. Slices of 250 µm think are transferred into an aCSF solution (in mM) : 124 NaCl, 2.5 KCl, 1.25 NaH₂PO₄, 2 MgCl₂, 2.5 CaCl₂, 10 glucose, 25 NaHCOs at 37°C for an hour then at room temperature.

Whole cell patch-clamp recordings were made using borosilicate electrodes (4-5 MΩ) filled with Biocytin (1%) and the following intracellular solution (in mM) : 135 Kgluconate, 3.8 NaCl, 1 MgCl₂, 10 HEPES, 0.1 EGTA, 0.4 Na₂-GTP, 2 Mg-ATP, pH 7.21, 291mOsm. Data were recorded using a MultiClamp 700B amplifier. Series and input resistances were measured on-line during recordings. Cells were rejected if series resistance changed by > 20 % during recordings. Action potentials were evoked by current steps injection (20 pA, 1 s) from -100 to +120 pA. Miniature synaptic currents were recorded at -60 mV in the presence of tetrodotoxine (1 µM). All recordings were analysed off-line using ClampFit (version 10.6).

### → In vivo extracellular recordings

Mice were first anaesthetized with urethane to maintain level of activity of monoaminergic system (20%) and placed on a stereotaxic frame (Unimécanique, Asnières, France). Segments L4-L5 of the spinal cord were exposed. For light stimulation, an optical fiber was lowered as close as possible from the nervous tissue allowing 577 nm light stimulation at 8mW/mm². Recording electrodes filled with NaCl 4% were built with glass capillaries from thin-walled borosilicate glass capillaries (outside diameter 1.5 mm; World Precision Instruments, Sarasota, FL, USA) and pulled using a puller (p-97; Sutter Instrument, Novato, CA, USA). Recordings were performed with an amplifier (DAM80, WPI, USA) connected to a CED1401 interface and recorded using Spike2 program (v7, CED, UK). Electric stimulations were performed with bipolar subcutaneous electrodes connected to an isolation box (isoflex, AMPI, Isr). Nociceptive field potentials were recorded using low pass filtered. Wide dynamic range (WDR) nature of the isolated units was assessed with electrical stimulation in the center of its receptive field and neurons with a fast (between 10 and 80ms) and slow (between 80 and 150ms) component were recorded using high pass filter.

Single unit recordings of identified WDR have been done in sham and 6-OHDA animals. First, we determined the minimal stimulation allowing slow response of the WDR. Then, we measured the response of the neurons to paw stimulation at 0.5, 1, 2, 3, 4 and 5mA with one stimulation every 15s. We then set the intensity as 3 times the threshold for optogenetic stimulations (continuous light for 1 min) and stimulated the paws again every 15s. Finally, windup was assessed with a wind-up protocol, i.e 15 stimulation at 1Hz still at an intensity corresponding to 3 times the threshold.

### Immunohistochemistry

### -> Fluorescence immunohistochemistry

Mice were kept deeply anaesthetized and then transcardially perfused with 50 mL of NaCl 0.9% Heparine and 50 ml of 4% PFA. Both brain and spinal cord were dissected and fixed overnight in 4% PFA, and then cryopreserved in 12.5% PBS sucrose. After being frozen in Tissue-Tek O.C.T, brain and spinal cord sections were cut transversely at 20 µm on a cryostat (Leica CM3050S) and kept in PBS1X Azide 0.2%.

After 3 bath wash of PBS 1X, sections were put free-floating in a solution of 0.1M PBS / BSA1% / triton 0,3% to permeabilize and saturate the slices. Slices were then bathed overnight at 4°C in solution composed of primary antibody (see table below) / PBS1X / BSA 1%. After being washed in PBS 1X, the secondary antibody (see table below) was added in 0.1M PBS and BSA 0.1%.

| **Fluorescent Antibody** | **Origin Species** | **Dilution** | **Reference** |
|---|---|---|---|
| *Primary antibody* | | | |
| Anti-PhosphoErk | Rabbit | 1/1000 | Biotechne NBP1-19924 |
| Anti-GFP | Chicken | 1/1000 | Aves GFP-1010 |
| Anti-TPH2 | Rabbit | 1/2000-1/5000 | Biotechne NB300-109 |
| Anti-DBH | Rabbit | 1/500 | Abcam |
| Anti-TH | Mouse | 1/2000 | Sigma-Aldrich |

| *Secondary antibody* | | | |
|---|---|---|---|
| Anti-Rabbit Alexa 488 | Goat | 1/500 | Thermo Fisher A11008 |
| Anti-chicken Alexa 488 | Goat | 1/500 | Thermo Fisher A-11039 |
| Anti-Rabbit Alexa 647 | Donkey | 1/500 | Thermo Fisher A21447 |
| Streptavidin Alexa488 or Alexa568 | Sheep | 1/500 | Invitrogen S11223 or S11226 |

### → DAB immunostaining

For TH (tyrosine hydroxylase) and DBH (dopamine beta-hydroxylase) quantification, we used DAB immunostaining. Slices are rinsed and permeabilized with PBS1X/BSA 2%/Triton 100X 0,3% solution for 30 min. Slices are then incubated overnight at 4°C with anti-TH rabbit (1 : 5000, Novus Biologicals, NB300-109) or anti-DBH rabbit (1/500, Abcam). Slices are incubated 30 min with a secondary antibody anti-rabbit VisUCyte (R&D systems, VC003-125) and then washed again. Finally, the reaction is revealed via the chromogen Diaminobenzidin (DAB) and slices are then mounted on gelatine slides.

We acquired the images of the striatum, SNc and A11 sections with the microscope Panoramic scan II 3DHISTECH. The images were analysed with ImageJ Fiji software (National Institutes of Health, New York, USA). Only mice with a lesion of >80% in the SNc and >90% in the striatum were included in the present study.

Grey level of the striatum TH staining is defined as the difference between the mean intensity of the pixels of the striatum (i.e. TH + staining) and the mean intensity of the pixels located in the cortex (i.e. background staining). Grey level of the DHSC/RVM is defined as the difference between the mean intensity of the pixels of the DHSC/RVM (i.e. TH + staining) and the mean intensity of the pixel located in blank surrounding area.

The number of TH cells in the SNc was obtained using the optical fractionator (Bastide et al. ([5])) unbiased stereological method (Leica DM6000B, Mercator Pro, ExploraNova, France). Immuno-labelled cells were counted by a blinded investigator on every 6 sections for the SNc (Counting frames: 60× 40 µm, Spacing: 120 × 180 µm, number of sections: 5). We counted the number of A11 TH+ neurons manually using Image J and its cell counting plugin.

### → Cell quantification

Images of the NRM of 5-HTCre*Ai9tdtomato animals were realised with the confocal microscope (Leica TCS SP5). The images were taken with 20x oil immersion objective. A stack of 20µm is imaged for each zone. TPH2, tdtomato or PhosphoERK positive cells were counted with the Cell Counting plugin of ImageJ software.

### Statistics

As the groups contain less than 30 elements, we used non-parametric tests. When 2 groups are compared, we used Wilcoxon test if the data are paired and Mann-Whitney test otherwise. To look at the effect of 2 factors on data, we used Two-Ways ANOVA test for parametric values or Kruskal Wallis for non-parametric values. Graphs and tests are done with GraphPad Prism software. Data are presented as Mean +/- SEM. Tests are considered are significant if p<0.05. *p<0.05; **p<0.01; ***p<0.001).

### RESULTS

6-OHDA injection induced DA neuron lesion associated with motor impairments, mechanical hypersensitivity and spinal hyperexcitability

We used a mouse model of hemiparkinsonism induced by unilateral 6-OHDA injection in the right MFB. This neurotoxin induced a subtotal lesion of DA neurons in the SNc and the ventral tegmental area (VTA) ipsilateral to the injection and a marked reduction in the ipsilateral DA fibers in the striatum compared to sham animals. Associated to this unilateral lesion, we observed a motor deficit, as shown by the decrease in the total distance travelled in the open field, and by the almost exclusive ipsilateral rotations in the cylinder test in 6-OHDA mice. We then evaluated mechanical sensitivity of the animals using Von Frey filaments. We show that DA depletion dramatically decreased mechanical withdrawal threshold in the ipsilateral and contralateral paws of the animals. In order to determine if the mechanical hypersensitivity is linked to spinal alteration, we recorded DHSC neurons that represent the first relay of nociceptive information. We first assessed the global nociceptive activity of the spinal cord by quantifying the local nociceptive field potentials (LFP) induced by noxious electrical peripheral stimulations. We show that LFP amplitude is increased in response to peripheral stimulations in 6-OHDA mice compared to sham. We then recorded wide dynamic range (WDR) neurons, which are located in the deep layers of the DHSC and represent a readout of the excitability of DHSC. WDR neurons responded to both innocuous (A-response) and noxious (C-response) peripheral stimulations. We focused here on the late nociceptive C-response following electrical stimulations applied to the plantar surface of the paw in the receptive field of WDR neurons. We measured the threshold for triggering a C-response of WDR neurons and we show that this threshold is significantly decreased in 6-OHDA mice compared to sham animals. The intensity-response curve for C-fibers is increased in 6-OHDA mice compared to sham animals. These results indicate that 6-OHDA injection induced hyperexcitability of the WDR neurons in the DHSC. To investigate whether hyperexcitability in 6-OHDA mice is due to altered intrinsic properties of WDR neurons, we studied the windup, which is a form of short-term central sensitization elicited by repetitive stimulations at 3 times the threshold intensity for C-fibers and at low frequency (1Hz). We found that 8/25 and 20/47 WDR elicit windup in sham and 6OHDA respectively (*p*=0.64) and that in 6-OHDA mice the windup curve is higher than in sham. However, there is a slight but not significant modification of the windup coefficient suggesting that increased windup curve is not due to change of intrinsic properties. To confirm this possibility, we normalized the windup curve to the 1^{st} response in order to focus on the amplification property. We show that amplification is strictly similar in 6-OHDA and sham mice suggesting that hyperexcitability in DHSC is due to alteration of the spinal network rather than intrinsic properties of WDR neurons.

Decreased DA fibers in the RVM and increased electrical activity of 5-HT neurons.

As DHSC neuronal activity is altered in our model, we focused on TH immunoreactivity in the DHSC of 6-OHDA mice and in the hypothalamic nucleus A11, considered as the DA nucleus projecting to the spinal cord (Koblinger et al. ([6])). TH immunoreactivity in the A11 and the DHSC remained unaffected, suggesting that DA fibers projecting to the DHSC are preserved in our 6-OHDA mouse model. We therefore investigated descending pain pathways that project to the DHSC and modulate nociceptive transmission, in particular the nucleus raphe magnus (NRM) that contains 5-HT neurons (Millan MJ ([7]); Aby et al. ([8]); Ganley et al. ([9])). We found that in NRM, 5-HT neurons are surrounded by dense TH-positive projections associated with close apposition in cell bodies and fibers. We found that these projections are both noradrenergic (NA) and dopaminergic (DA). However, DA fibers are significantly reduced in the NRM of 6-OHDA mice. NA fibers remained unaffected in part because NA neurons were protected by i.p. injection of desipramine in our model (see method). We then assessed tissue levels of NA and 5-HT in the lumbar spinal cord of sham and 6-OHDA mice, using the HPLC coupled to electrochemical detection. Biochemical analysis showed that NA levels were unchanged in 6-OHDA animals compared with sham animals (**Fig. 1A**, test, *p*=0.18). On the other hand, levels of 5-HT and its metabolite 5-HIAA were significantly increased (**Fig. 1B-C**, 5-HT: *p*<0.05; 5-HIAA: *p*<0.05). Interestingly, the 5-HT/5-HIAA ratio remained unchanged (**Fig.1D**, *p*=0.24). These results suggest that DA depletion is associated to an increased activity of 5-HT neurons.

To confirm this hypothesis, we investigated the expression of phosphorylated extracellular signal-regulated kinases (pERK), a marker of neuronal activity, in 5-HT neurons of the NRM. Using *epet-cre::Ai9 td tomato* transgenic mice (Aby et al. ([8]); Scott et al. ([10])), we compared the expression of pERK in NMR 5-HT neurons of 6-OHDA and sham animals. We first quantified the number of tomato positive neurons of the NRM (**Fig. 1E**) and found no difference between sham and 6-OHDA mice (*p*=0.5). Then, using immunohistochemistry against pERK we show that the number of pERK+ neurons in the NRM significantly increased in 6-OHDA mice compared to sham animals (**Fig. 1F**, *p*<0.001). This is due notably to an increased number of 5-HT neurons positive to pERK (**Fig.1G**, *p*<0.01). Finally, if we restrict quantification to NMR 5-HT neurons, we show that the proportion of 5-HT neurons positive to pERK in 6-OHDA mice is significantly higher than in sham animals (**Fig. 1H** *p*<0.01). We performed *ex vivo* recordings of 5-HT neurons, using the patch-clamp technique of NRM slices from both sham and 6-OHDA *epet-cre::Ai9 td tomato* mice (**Fig. 2A**). We investigated both the intrinsic properties and the synaptic connections of 5-HT neurons. We show that resting potential, action potential amplitude and shape of 5-HT neurons in the NRM are not modified in 6-OHDA mice compared to sham animals (resting potential;-57.3±1.2mV sham, -61±1.5mV 6-OHDA, *p*=0.14; amplitude; 53±2.2mV sham and 59.5±1.5mV 6-OHDA, *p*=0.40 and shape; 1.1±0.04ms sham and 1.2+0.07ms 6-OHDA, p=0.40). However, we found a significant increase in the membrane resistance (**Fig. 2B**, *p*<0.05), an increase in membrane time constant (**Fig. 2C**, *p*<0.01), and a decrease of action potential threshold (**Fig. 2D**, *p*<0.01). These results suggest that DA depletion increased the excitability of 5-HT neurons in line with their increased activity. Furthermore, we measured the miniature excitatory synaptic currents (mEPSCs) of 5-HT neurons (**Fig. 2E**). In 6-OHDA mice, we observed a significant increase in the amplitude of the mEPSCs (**Fig. 2F,** *p*<0.05) as well as a significant decrease in inter-event intervals (**Fig. 2G,** *p*<0.05) as compared to sham conditions. These results show an increased excitatory barrage onto 5-HT neurons that are also more excitable. Altogether, these results strongly suggest that 5-HT neurons of the NRM are hyperactive in the context of PD.

Inhibition of 5-HT descending pathway in DA depleted animals reversed mechanical hypersensitivity.

We next assessed the role of 5-HT descending pathway in mechanical pain hypersensitivity and spinal hyperexcitability in 6-OHDA mice. We injected a cre-dependent adenovirus (AAV-dio-ARchT-GFP) in NRM of *epet-cre* mice allowing the specific expression of Archaerhodopsin (ArchT), an inhibitory opsin acting as a proton-pump, in 5-HT neurons (**Fig. 3A**). We used a group of *epet-cre* mice infected with an AAV-dio-GFP as a control group. We implanted optical fibers necessary for opsin activation above dorsal surface of the spinal cord to specifically inhibit 5-HT descending fibers (see method and Aby et al. ([8])). Optogenetic inhibition of the 5-HT descending pathways significantly increased mechanical withdrawal threshold of 6-OHDA mice expressing ArchT but not GFP alone **(****Fig. 3B-C****,** *p*<0.001 for ArchT and *p*=0.75 for GFP). We then investigated the effect of the optogenetic inhibition on spinal hyperexcitability (**Fig. 3D**) and we show that optogenetic inhibition of 5-HT descending pathway decreased the global size of nociceptive LFP of the DHSC (**Fig. 3E-F,** *p*<0.01 for ArchT and *p*=0.48 for GFP). These results show that inhibition of 5-HT descending pathway from the NRM is able to reverse dorsal horn hyperexcitability and mechanical hypersensitivity.

5-HT_{2A} and 5-HT₃ receptors are involved in mechanical hypersensitivity in 6-OHDA mice.

Finally, we addressed the question of the 5-HT receptors involved in such nociceptive hypersensitivity. We performed intrathecal injections of 5-HT receptor antagonists and evaluate mechanical sensitivity in 6-OHDA mice. The selective 5-HTR_{2A} antagonist MDL11939 partially reversed mechanical withdrawal threshold (**Fig. 3G**, *p*<0.05). The 5-HTR₃ antagonist granisetron also significantly increased the mechanical threshold (**Fig. 3G,** *p*<0.001). Finally, we tested the effect of simultaneous blockade of 5-HT_{2A} and 5-HT₃ receptors. The combined intrathecal injection of granisetron and MDL11239 significantly increased the mechanical threshold to a level close to that of sham animals, suggesting a synergistic effect of the two antagonists (**Fig. 3G**, *p*<0.01). To confirm this hypothesis, we plotted the different combinations and compared the difference with the values of the sham animals (**Fig. 3H**). We found that combination of granisetron and MDL11239 induced an increase in mechanical threshold that was not different from that of the sham condition (*p*=0.99).

### DISCUSSION

In the present study, we demonstrated that 5-HT neurons from the NRM exert a descending facilitation on nociceptive transmission in the 6-OHDA mouse model of PD. We show that DA neuron lesion induced mechanical hypersensitivity associated with dorsal horn neuron hyperexcitability. This hyperexcitability is associated with a loss of TH fibers and hyperactivity of 5-HT neurons in the NRM. Selective optogenetic silencing of 5-HT neurons reversed the pathological mechanical hypersensitivity and spinal hyperexcitability. Finally, we show that the pathological hypersensitivity was reversed by the synergistic blockade of 5-HT_{2A} and 5-HT₃ receptors.

### Unilateral dopamine depletion induced spinal hypersensitivity

We used a model of hemiparkinsonism obtained by the unilateral injection of 6-OHDA in the MFB of adult mice, inducing a dramatic unilateral lesion of DA neurons in the SNc and fibers in the striatum (Luan et al. ([11]); Tang et al. ([12]). By contrast, we show that A11 DA neurons were not damaged as were the DA fibers in the spinal cord. Associated to the loss of DA neurons, we observed motor impairments as shown by unilateral rotations in the cylinder test and reduced locomotor activity in the open field test. Interestingly, following unilateral lesion of DA nigro-striatal pathway, we observed bilateral allodynia as the mechanical threshold in the hind paw decreased. This result is in line with previous studies carried out in mice and patients.

We also show that unilateral lesion induced a dorsal horn neuron (DHN) hyperexcitability. We evaluated the activity of DHN that respond to the stimulation of both non-nociceptive and nociceptive peripheral fibers. These neurons are called wide dynamic range neurons (WDR) and are considered as one of the main outputs from the DHSC. Changes in WDR excitability is a good indicator of sensitization of the DHSC, a phenomenon occurring following injury of peripheral or central nervous system. We show that WDR increased their responsiveness to nociceptive inputs, which was present for all stimulation intensities. We and others have previously observed an increase in the excitability and an alteration in the intensity-response of WDR neurons in the DHSC in rats following unilateral injection of 6-OHDA into the MFB suggesting that spinal excitability is a main feature of 6OHDA-induced nociceptive hypersensitivity in mammals. Finally, we found no modification of the windup, a form of short-term plasticity expressed by WDR suggesting that sensitization capabilities of 6-OHDA mice depend on spinal network rather than on WDR intrinsic properties. This result is different from what we observed in 6-OHDA rats where windup amplitude was increased in WDR neurons. However, this discrepancy may be explained by the different anesthetics used in the two studies. In rats, electrophysiological recordings were made under isoflurane anesthesia, whereas in mice we used urethane. This was justified by the fact that isoflurane inhibits the activity of 5-HT neurons.

6-OHDA-induced dopamine neuron lesion increased the activity of 5-HT neurons of the NRM.

Motor impairments as well as nociceptive hypersensitivity and spinal cord neurons hyperexcitability are a consequence of the loss of dopaminergic nigro-striatal pathway. We investigated the involvement of the serotonergic system, in particular that of the descending pathway from the NRM to the spinal cord. This pathway is known to be involved in the control of nociception and consequently of pain. First, we explored the anatomical organization of catecholaminergic fibers in the NRM and found that 5-HT neurons are surrounded by both dopaminergic and noradrenergic projections. Moreover, TH+ labelling in the NRM is presumably less dense in our model of hemiparkinsonism while the DBH staining remained similar. This could be due to a reduction of DA fiber's density in the NRM of 6-OHDA mice, a hypothesis pending quantitative analysis. We observed an increase in 5-HT levels in the DHSC while the dynamic of 5-HT degradation remained unchanged, and the 5-HT/5HIAA ratio was stable. Together, this suggests that the increase in 5-HT levels is not due to a deficiency in the pathway degradation but rather to an increase in the activity of 5-HT neurons. Furthermore, we show that the number of 5-HT neurons in the NRM is not modified in 6-OHDA mice and that the number of neurons expressing pERK, a marker of neuronal activity, is increased in this area and particularly in 5-HT neurons. These results are not in agreement with those of two previous studies showing a decrease in spinal 5-HT levels associated with a loss of 5-HT neurons in B3 nucleus in rats (Campos et al. ([13]); Wang et al. ([14])). However, in these studies, 6-OHDA was injected either bilaterally into the SNc or unilaterally into the striatum of rats, which may explain the differences observed. On the other hand, 5-HT neurons in the NRM do not send fibers either to the cerebral cortex or to subcortical areas, thereby preventing direct contact of NMR fibers with 6-OHDA into the MFB and loss of these 5-HT neurons. Moreover, using patch clamp recordings of identified 5-HT neurons of NRM, we show that 5-HT neurons are more excitable and receive an increased barrage of mEPSCs in 6-OHDA compared to sham condition. These results show that 5-HT neurons in 6-OHDA mice receive more excitatory inputs that confirm the increased activity of 5-HT neurons of the NRM. Taken together, our results demonstrate that the severe lesion of DA neurons induces hyperactivity of 5-HT system in the NRM.

Selective optogenetic inhibition of NRM-Spinal 5-HT descending pathway controls nociceptive transmission in the context of PD.

We show that 5-HT neuron activity is increased and this increase is associated with spinal hyperexcitability and mechanical pain hypersensitivity. We used an optogenetic approach to express ArchT opsin in NRM 5-HT-neurons. Activation of ArchT by green light is known to induce proton efflux, resulting in strong hyperpolarization. To specifically target 5-HT fibers projecting to the spinal cord, we implanted an optical fiber above the DHSC to illuminate descending fibers. In 6-OHDA 5-HT-cre mice, we show that optogenetic inhibition of 5-HT fibers increased mechanical threshold and generated a decrease in hyperexcitability of DHSC neurons.

These results are in agreement with previous studies showing the involvement of 5-HT descending pathway in nociceptive transmission in different pain models other than Parkinson models (Aby et al. ([8]); Ganley et al. ([9]); Cai et al. ([15])). Furthermore, in our recent study we showed that activation of NRM 5-HT neurons generated hyperalgesia (Aby et al. ([8])) and that optogenetic manipulation of NRM 5-HT neurons mediated mechanical analgesia through activation of inhibitory interneurons in a model of peripheral nerve injury in 5-HT-cre mice (Aby et al. ([8])).

5-HT_{2A} and 5-HT₃ receptors mediated spinal hyperexcitability in 6-OHDA mouse model of hemi-parkinsonism.

To directly associate spinal hyperexcitability and 5-HT descending pathways in our 6-OHDA mouse model of hemiparkinsonism, we used a pharmacological approach targeting 5-HT receptors. Our results show that at least two 5-HT receptors are involved in mechanical allodynia. 5-HT is associated with 7 different receptors 5-HT1-7 and at least 15 subtypes (Millan MJ ([7]). A recent study identified at least 12 subtypes involved in the DHSC responses to 5-HT receptor manipulation (Bourgoin et al. ([16])). We also showed that the 5-HT descending pathway of the NRM exerted an analgesic action and that this same pathway has become pronociceptive in pathological context of neuropathic pain (Aby et al. ([8])). We show here that brainstem 5-HT system is hyperactive following DA depletion leading to a pronociceptive effect of 5-HT descending pathway participating in nociceptive hypersensitivity. Facilitation of the 5-HT descending pathway has been observed in various pathological contexts, including inflammation and neuropathic pain, and is thought to be associated with 5HT_{2A} and 5-HT₃ receptor subtypes (Bardin et al. ([17])). Here we show that inhibition of the descending pathway projecting from the NRM to the spinal cord is analgesic and that spinal hypersensitivity can be blocked with antagonists of both 5-HT_{2A} and 5-HT₃ receptors. Furthermore, we show that these antagonists individually exert a significant analgesic effect, but their combination almost completely reversed mechanical allodynia in 6-OHDA mice. This result suggests a synergistic action of the blockade of 5-HT_{2A}R and 5-HT₃R on mechanical allodynia. This synergistic effect could be due to the action of these two receptors in different neuronal subpopulation at the DHSC (Liu et al. ([18])).

In conclusion, our results show that 6-OHDA-induced DA depletion in mice, induced a spinally mediated pain hypersensitivity associated with an impairment of the of 5-HT descending pathway due to hyperactivity of NRM 5-HT neurons. This hyperactivity participates in mechanical pain by promoting nociceptive transmission in the spinal cord at least involving 5-HT_{2A}R and 5-HTsR. These results show that therapeutic approaches aiming at downregulating the activity of NRM 5-HT neurons or pharmacologically blockade of spinal 5-HT_{2A}R and 5-HT₃R are pertinent in the treatment of pain in PD.

### Reference List

1. Politis M, Niccolini F. Serotonin in Parkinson's disease. Behav Brain Res. 2015;277:136-145. doi: 1 0.1 016/j.bbr.2014.07.037.
2. Christensen AJ, Iyer SM, François A, et al. In Vivo Interrogation of Spinal Mechanosensory Circuits. Cell Rep. 2016;17(6):1699-1710. doi:10.1016/j.celrep.2016.10.010.
3. Boix J, Padel T, Paul G. A partial lesion model of Parkinson's disease in mice--characterization of a 6-OHDA-induced medial forebrain bundle lesion. Behav Brain Res. 2015;284:196-206. doi:10.1016/j.bbr.2015.01.053.
4. Bonin RP, Bories C, De Koninck Y. A simplified up-down method (SUDO) for measuring mechanical nociception in rodents using von Frey filaments. Mol Pain. 2014;10:26. doi:10.1186/1744-8069-10-26.
5. Bastide MF, Dovero S, Charron G, et al. Immediate-early gene expression in structures outside the basal ganglia is associated to I-DOPA-induced dyskinesia. Neurobiol Dis. 2014;62:179-192. doi:10.1016/j.nbd.2013.09.020.
6. Kathrin Koblinger, Tamás Füzesi, Jillian Ejdrygiewicz, et al. Characterization of A11 Neurons Projecting to the Spinal Cord of Mice. Plos One. October 24, 2014, https://doi.org/10.1371/journal.pone.0109636.
7. Millan MJ. Descending control of pain. Prog Neurobiol. Published online 2002:120.
8. Aby F, Lorenzo LE, Grivet Z, et al. Switch of serotonergic descending inhibition into facilitation by a spinal chloride imbalance in neuropathic pain. Sci Adv. 2022;8(30):eabo0689. doi:10.1126/sciadv.abo0689.
9. Ganley RP, De Sousa MM, Werder K, et al. Targeted anatomical and functional identification of antinociceptive and pronociceptive serotonergic neurons that project to the spinal dorsal horn. eLife. 2023;12:e78689. doi:10.7554/eLife.78689.
10. Scott MM, Wylie CJ, Lerch JK, et al. A genetic approach to access serotonin neurons for in vivo and in vitro studies. Proc Natl Acad Sci U S A. 2005;102(45):16472-16477. doi:10.1073/pnas.0504510102.
11. Luan Y, Tang D, Wu H, et al. Reversal of hyperactive subthalamic circuits differentially mitigates pain hypersensitivity phenotypes in parkinsonian mice. Proc Natl Acad Sci. 2020;117(18):10045-10054. doi:10.1073/pnas.1916263117.
12.25. Tang DL, Luan YW, Zhou CY, Xiao C. D2 receptor activation relieves pain hypersensitivity by inhibiting superficial dorsal horn neurons in parkinsonian mice. Acta Pharmacol Sin. 2021;42(2):189-198. doi:10.1038/s41401-020-0433-3.
13. Campos ACP, Berzuino MB, Hernandes MS, Fonoff ET, Pagano RL. Monoaminergic regulation of nociceptive circuitry in a Parkinson's disease rat model. Exp Neurol. 2019;318:12-21. doi:10.1016/j.expneurol.2019.04.015.
14.34. Wang CT, Mao CJ, Zhang XQ, et al. Attenuation of hyperalgesia responses via the modulation of 5-hydroxytryptamine signalings in the rostral ventromedial medulla and spinal cord in a 6-hydroxydopamine-induced rat model of Parkinson's disease. Mol Pain. 2017;13. doi:10.1177/1744806917691525.
15. Cai YQ, Wang W, Hou YY, Pan ZZ. Optogenetic Activation of Brainstem Serotonergic Neurons Induces Persistent Pain Sensitization. Mol Pain. 2014;10:1744-8069-10-70. doi:10.1186/1744-8069-10-70.
16. Bourgoin S, Gautier A, Hamon M. Le yin et le yang de la sérotonine : un Janus dans les contrôles de la douleur. Douleur Analgésie. 2017;30(1):35-56. doi:10.1007/s11724-017-0486-3.
17. Bardin L. The complex role of serotonin and 5-HT receptors in chronic pain. Behav Pharmacol. 2011;22(5-6):390-404. doi: 10.1097/FBP.0b013e328349aae4.
18.39. Liu QQ, Yao XX, Gao SH, et al. Role of 5-HT receptors in neuropathic pain: potential therapeutic implications. Pharmacol Res. 2020; 159: 1 04949. doi:10.1016/j.phrs.2020.104949.

## Claims

1. A combination comprising at least one 5-HT_{2A} receptor antagonist and at least one 5-HT₃ receptor antagonist.

2. A combination according to claim 1, wherein said at least one 5-HT_{2A} receptor antagonist is selected in the group consisting of glemanserin, ketanserin, altanserin, volinanserin, ritanserin, clozapine, loxapine, olanzapine, aripiprazole, quetiapine, ziprasidone, asenapine, amitriptyline, clomipramine, cyproheptadine, doxepin, eplivanserin, etoperidone, haloperidol, hydroxyzine, iloperidone, methysergide, mianserine, mirtazapine, nefazodone, pimavanserin, pizotifen, trazodone, yohimbine, spiperone, pirenperone and risperidone.

3. A combination according to claim 1 or 2, wherein said at least one 5-HT₃ receptor antagonist is selected in the group consisting of granisetron, ondansetron, tropisetron, alosetron, dolasetron, palonosetron, ramosetron, memantine, metoclopramide and vortioxetine

4. A combination according to claim 1, wherein said 5-HT_{2A} receptor antagonist is glemanserin and said 5-HT₃ receptor antagonist is granisetron.

5. A combination according to claim 1, wherein said 5-HT_{2A} receptor antagonist is ketanserin and said 5-HT₃ receptor antagonist is granisetron.

6. A pharmaceutical composition comprising at least one 5-HT_{2A} receptor antagonist and at least one 5-HT₃ receptor antagonist as defined in claims 1 to 5, and optionally at least one other active ingredient.

7. A pharmaceutical composition according to claim 6, wherein said at least one active ingredient is selected in the group consisting of L-Dopa, a dopamine agonist such as apomorphine, pramipexole, ropinirole or rotigotine, antidepressants such as serotonin noradrenaline reuptake inhibitors (SNRI) or tricyclic antidepressants (TCA), and GABAergic agents such as pregabaline or gabapentine.

8. A kit of parts comprising at least one 5-HT_{2A} receptor antagonist and at least one 5-HT₃ receptor antagonist as defined in claims 1 to 5, as a combined preparation for simultaneous, separate or sequential use.

9. The combination according to any one of claims 1 to 5, or the pharmaceutical composition according to claims 6 or 7, for medical use.

10. The combination according to any one of claims 1 to 5, or the pharmaceutical composition according to claim 6 or 7, for use in the treatment or prevention of non-motor component of Parkinson's disease.

11. The combination according to any one of claims 1 to 5, or the pharmaceutical composition according to claim 6 or 7, for use according to claim 10, wherein said non-motor component of Parkinson's disease is increased chronic pain.
